# EUROPEAN PATENT APPLICATION

(11) **EP 4 667 021 A1**
(43) Date of publication of application: **24.12.2025**
(21) Application number: 24382667.4
(22) Date of filing: 20.06.2024
(51) Int. Cl.: A61L 9/014, B01D 53/04, F24F 8/108

(54) **AIR FILTER AND ASSOCIATED LABORATORY CABINET**

(71) Applicant: Diantech Solutions, S.L., 08830 Sant Boi de Llobregat (Barcelona) (ES)
(72) Inventor: Pallarés Aranda, Jordi, 08830 SANT BOI DE LLOBREGAT (BARCELONA) (ES); Himicevs, Daniels, 08830 SANT BOI DE LLOBREGAT (BARCELONA) (ES); Canals Nieto, Alex, 08830 SANT BOI DE LLOBREGAT (BARCELONA) (ES)
(74) Representative: Clarke, Modet y Cía., S.L.

(57) **Abstract**

Air filter, comprising at least one housing (2) with an inner space (S) configured to contain an adsorbent (C) and featuring; a suction side (3) with one or more suction openings (31) allowing the entry of an air flow (F) into the filter (1); and a extraction side (4) with one or more extraction openings (41) allowing the air flow (F) to exit out of the filter (1). The inner space (S) is delimited by end deflecting means (5), configured to redirect the air flow (F) towards at least one extraction opening (41), wherein said deflecting means (5) are arranged at a first longitudinal end (1L₁) and/or at a second longitudinal end (1L₂) of the filter (1); and/or at a first transverse end (1T₁) and/or at a second transverse end (1T₂) of the filter (1).

## Description

### Field of the Invention

The present invention refers to an air filter, especially of the type that uses adsorbents such as carbon (chemically activated or not), zeolite or alumina, being particularly suitable for application in laboratory cabinets, such as gas filtration cabinets. The present invention also applies to a laboratory cabinet associated to the above mentioned filter.

### Background of the Invention

Air filters of the indicated type, such as those using active carbon adsorbents from coconut shell, for example, have a large proportion of micropores, and are the most suitable for adsorbing gaseous pollutants.

There are currently several types of known filters, which can be combined between them according to the work needs. For example:
- A: General purpose filter, especially suitable for organic vapors such as ketones, ethers, xylene, alcohols, etc.
- BE: For inorganic acid fumes such as: H₂SO₄, HCl, HNOs, as well as volatile sulfur compounds such as H₂S, SOs, etc.
- F: For formaldehyde and formalin fumes, and their derivatives. It can also be used for other organic compounds.
- K: For NH₃ and amine fumes. It can also be used for other organic compounds.

The adsorption phenomenon initially takes place in a small section of the filter bed, known as the mass transfer zone or MTZ. As the MTZ reaches its capacity limit (saturation) it progressively moves through the thickness of the filter, until it reaches the top. It is then said that the breakthrough has been reached, by observing a gradual increase in the concentration of the pollutant gas until reaching a total saturation of the filter.

As for the gas filtration cabinets, it should be noted that they do not require a coupled extraction equipment, since they have a filtration system to retain gases and polluting vapors generated inside the cabinet, thus constantly renewing the air in the laboratory and providing many advantages over conventional extraction hoods:
- The pollutants are not released into the environment but are instead retained in the filter.
- They do not require the installation of outdoor exhaust ducts.
- Its small size and weight, as well as the absence of exhaust duct systems, make it possible to replace them it in the event of a change of needs of the laboratory.
- The aspirated air is not expelled to the environment but indeed recirculated back into the laboratory, free of contaminants. This does not increase the consumption of air conditioning to replace the extraction loss.

To obtain maximum filtration efficiency, the following vital parameters should be considered in the design and construction of recirculating filtration cabinets with air filters of the indicated type.

Adsorption is a process of physicochemical nature in which molecules of a gas or liquid, called adsorbate, interact and adhere to the surface of a solid, called adsorbent or substrate. This phenomenon is widely used in the purification of liquid or gaseous streams. Gas phase applications include air odor removal, solvent recovery for reuse, respiratory protection, etc.

There are different types of adsorbents such as activated carbon, silica gel, alumina, zeolites and synthetic resins. The most important characteristic of an adsorbent material is its high porosity, which gives it a high specific surface area. Inside this distribution of pores of different sizes, the gaseous molecules are adsorbed and eventually condense and are retained.

Activated carbon, for example, is a carbonized material that has undergone an activation process with the purpose of increasing its porosity. Activated carbon has a series of characteristics that convert it in a highly valued adsorbent material:
- Its manufacturing process ensures a great development of its active surface.
- It has the physical properties necessary to ensure a good mechanical resistance.
- The cost of the manufacturing process and the raw material is accessible.

The retention capacity of the activated carbon for a given gas depends on the characteristics of the adsorbent (chemical composition, pore size and distribution, surface area, and particle size), the adsorbate (chemical composition, molecular size, boiling point and polarity), the concentration of the adsorbate in the liquid phase and the characteristics of the phase (pH, temperature, vapor pressure and humidity).

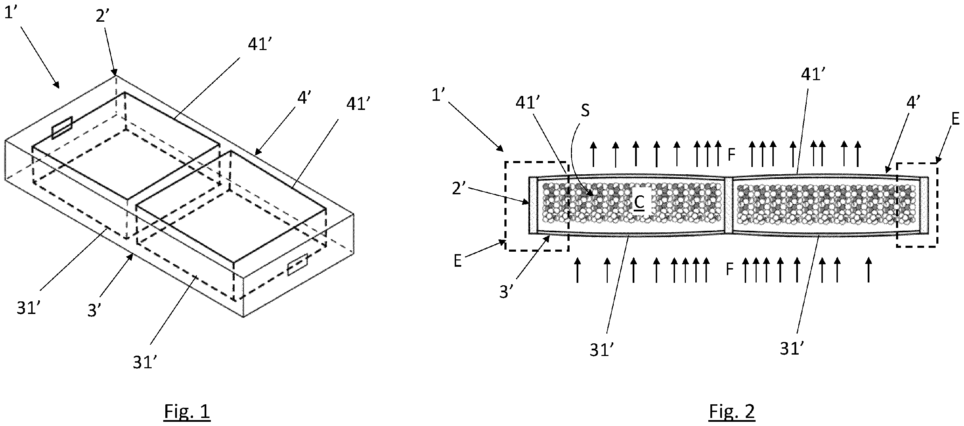

Conventional air filters usually consist of a housing configured to contain a compact filling of an adsorbent, such as activated carbon. This housing has an air aspiration side and an extraction side. The suction side has a distribution of suction openings that define a suction area that allows an air flow into the filter. The extraction side has a distribution of extraction openings that define an extraction area that allows airflow to go out from the filter. The suction openings on the suction side are distributed exactly the same and/or symmetrical to the extraction openings on the extraction side, as shown in Figures 1 and 2.

Thus, since the cabin motor is normally located on the extraction side and in the central part of the filter, most of the air flow passes through the central part of the filter, without passing through the ends or side areas of the filter, therefore seeking the path of least pressure drop.

Therefore, in conventional activated carbon filters such as the one in Figures 1 and 2, a large amount of unused activated carbon is discarded because the breaking point is reached prematurely, usually in the central part of the filter, and not homogeneously. This leads to a replacement of the filter even when much of the activated carbon is still in perfect condition to continue with the adsorption.

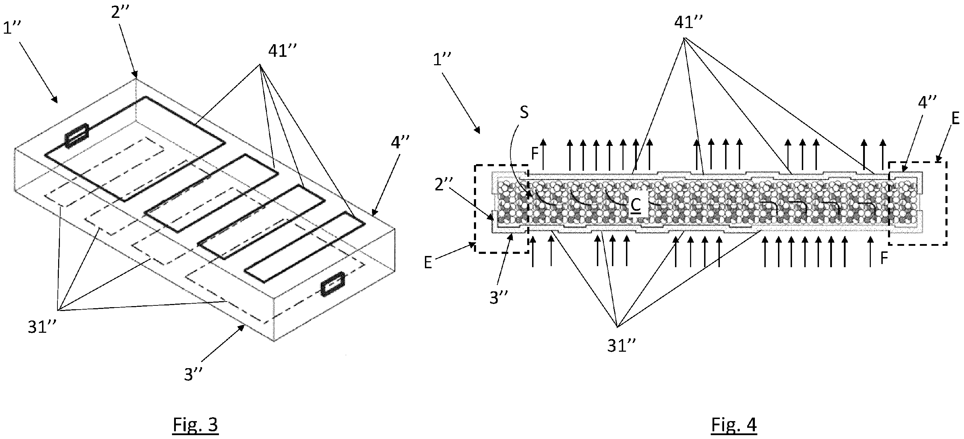

Document WO2024032965A1 refers to an air filter that allows a greater use of the adsorbent, thus maximizing the efficiency of the filter, extending its useful life with product retention capacities greater than those of conventional air filters. Such a filter is characterized by the fact that the suction openings of the air aspiration side are distributed symmetrically inverse to the extraction openings of the extraction side to redirect the air flow inside the filter, as shown in Figures 3 and 4.

The air filter configuration of document WO2024032965A1 allows directing the air flow through the adsorbent filler in order to increase the contact area between the adsorbent and the adsorbate; and to decrease its velocity so that the contact time between the filter coating and the air flow is much longer, which results in a higher optimization of the adsorbent, i.e., it considerably increases the residence time of the flow within the filter bed.

Despite the improvements of the air filter of document WO2024032965A1, both in this and in the previously described conventional filters there is the formation of air-tight zones inside the filter, where the adsorbent is hardly used, since the air flow does not reach them. These areas are particularly noticeable at the lateral ends, both at the longitudinal and transverse ends.

The present invention refers to an air filter specially designed to ensure that the entire adsorbent is exposed to the air flow, thus improving its use. By improving the contact efficiency of the air and the adsorbent, less adsorbent can be used without compromising the filtration capacity of the system. This reduces material costs and makes the filter lighter and easier to handle.

### Description of the Invention

The air filter of the present invention comprises at least one housing with an interior space configured to contain an adsorbent and featuring:
- a suction side with one or more suction openings that allow an air flow into the filter; and
- an extraction side with one or more extraction openings that allow the air flow out of the filter.

The filter is characterized in that the interior space is bounded by end deflecting means configured to redirect the air flow towards at least one extraction opening, wherein said deflecting means are arranged in:
- a first longitudinal end and/or at a second longitudinal end of the filter; and/or in
- a first transverse end and/or at a second transverse end of the filter.

The absorbent is therefore contained only within the inner space of the housing, the end deflecting means occupying a free absorbent zone. So that the longitudinal and/or transverse ends are free of absorbent.

The end deflecting means are designed to direct and distribute the air flow evenly across the adsorbent, eliminating areas where the air could become stagnant and not interact with the adsorbent material. The extreme deflecting means prevent the formation of air-tight zones inside the filter, where the adsorbent is not effectively used. This ensures that the entire adsorbent is exposed to the air flow, improving its utilization. By improving the contact efficiency of the air and the adsorbent, less adsorbent can be used without compromising the filtration capacity of the system. This reduces material costs and makes the filter lighter and easier to handle.

According to a preferred embodiment, the end deflecting means comprise:
- a first transverse baffle arranged along the first longitudinal end of the filter; and/or
- a second transverse baffle arranged along the second longitudinal end of the filter.

The suction openings and the extraction openings are arranged parallel to the first transverse baffle and/or the second transverse baffle.

According to another preferred embodiment, the end deflecting means comprise:
- a first longitudinal baffle arranged along the first transverse end of the filter; and/or
- a second longitudinal baffle arranged along the second transverse end of the filter.

The suction openings and the extraction openings are arranged parallel to the first longitudinal baffle and/or the second longitudinal baffle.

This arrangement of the openings reduces the path that the air flow must take through the filter, since it circulates across the width of the filter and not along its length. As a result, the air flow encounters less resistance as it passes through the adsorbent, thus reducing the overall pressure drop of the system.

Preferably, the end deflecting means are arranged:
- close to the suction side contiguous to a suction opening; and/or
- close to the extraction side adjacent to an extraction opening.

Located on the suction side, the end deflecting means are positioned just after the suction openings. Its main function is to guide the incoming air flow so that it is evenly distributed across the adsorbent. This ensures that the entire volume of air passes through the adsorbent material, maximizing contact and adsorption efficiency.

Located on the extraction side, the end deflecting means are positioned just before the extraction openings. Its function is to redirect the outgoing air flow, ensuring that a constant, turbulence-free flow is maintained. This helps maintain system efficiency and ensures that the air has adequately passed through the adsorbent before leaving the filter.

Preferably, the end deflecting means form an integral part of the housing, being part of its structure, or independent elements attached to it, or combinations of both. So, filter manufacturing can be done by various industrial processes; molding, assembly, additive manufacturing, etc.

Preferably, the end deflecting means have at least one end deflecting surface in contact with the adsorbent, which has a straight or curved configuration and which is arranged obliquely to the suction side and to the extraction side. Both straight and curved configurations, and especially the curved one, favor the aerodynamics of the activated carbon and the demolding process of the part.

According to a preferred embodiment, the interior space is further delimited by intermediate deflecting means, configured to redirect the air flow towards at least one exhaust opening, wherein said intermediate deflecting means are arranged between:
- the first longitudinal end and the second longitudinal end of the filter; and/or between
- the first transverse end and the second transverse end of the filter.

The absorber is thus contained only within the inner space of the housing, the intermediate deflecting means occupying an absorber-free zone between the longitudinal ends and/or between the transverse ends.

The intermediate deflecting means are also designed to direct and distribute the air flow evenly across the adsorbent, eliminating areas where air could become stagnant and not interact with the adsorbent material. The intermediate deflector media prevent the formation of air-tight zones inside the filter, where the adsorbent is not effectively used. This ensures that the entire adsorbent is exposed to the air flow, improving its utilization. By improving the contact efficiency of the air and the adsorbent, less adsorbent can be used without compromising the filtration capacity of the system. This reduces material costs and makes the filter lighter and easier to handle.

Preferably, the intermediate deflecting means comprise a first intermediate baffle proximal to the suction side and contiguous to a suction opening.

Located on the suction side, the intermediate deflecting means are positioned just after the suction openings. Its main function is to guide the incoming air flow so that it is evenly distributed across the adsorbent. This ensures that the entire volume of air passes through the adsorbent material, maximizing contact and adsorption efficiency.

Preferably, the intermediate deflecting means comprise a second intermediate baffle proximate to the extraction side and contiguous to an extraction opening.

Located on the extraction side, the intermediate deflecting means are positioned just before the extraction openings. Its function is to redirect the outgoing air flow, ensuring that a constant, turbulence-free flow is maintained. This helps maintain system efficiency and ensures that the air has adequately passed through the adsorbent before leaving the filter.

Preferably, the intermediate deflecting means form an integral part of the housing, being part of its structure, or independent elements attached to it, or combinations of both. So, filter manufacturing can be done by various industrial processes; molding, assembly, additive manufacturing, etc.

Preferably, the intermediate deflecting means have at least one intermediate deflecting surface in contact with the adsorbent, which has a straight or curved configuration and which is arranged obliquely to the suction side and to the extraction side. Both straight and curved configurations, and especially the curved one, favor the aerodynamics of the activated carbon and the demolding process of the part.

Preferably, the suction openings of the suction side are distributed inversely, alternately or symmetrically reversed to the extraction openings of the extraction side to redirect the air flow inside the filter.

The suction side therefore has suction openings that allow the air flow to enter through them, arranged between suction separation zones that prevent the passage of air flow. Similarly, the extraction side has extraction openings that allow airflow to escape through these, arranged between extraction separation zones that prevent airflow.

Thus, by arranging this combination of openings and separation areas in reverse on one side and the other face of the filter, it is possible to redirect the air flow inside, making better use of the adsorbent. The reverse distribution of openings allows redirecting the flow of circulating air through the filter filler, favoring the circulation of air flow through the filter in its longitudinal or transversal direction. This results in an increased pressure drop perpendicular to the filter, which prevents the air from circulating perpendicular to the filter, thus making it an unfavorable path.

Consequently, this fact causes a decrease in the air flow exit velocity. This allows to considerably increase the residence time of the flow inside the filter bed by achieving a slower air flow. This fact implies that the contact time between the adsorbent filling of the filter and the flow is much longer, at the same time that there is a circulation throughout the adsorbent bed contained in the interior space, which translates into a greater optimization, performance and/or use of the adsorbent, in addition to avoiding the presence of airtight air zones where the adsorbent is not properly used thanks to the deflecting means.

Preferably, the housing is formed by two half bodies, where each of them comprises the suction side or the extraction side, facing each other and joined perimetrally.

This union is preferably a thermal union or with addition of material, such as polypropylene, for better compatibility. Preferably, the adsorbent is filled after the junction, using side holes to introduce the adsorbent particles. The housing is preferably waterproof.

It is preferable that the casing or housings that make up the filter are made of materials with high chemical resistance. This material must be able to withstand prolonged exposure to various airborne chemical contaminants without degradation or loss of structural integrity. Polypropylene is a suitable material due to its resistance to many acids, alkalis and solvents.

In addition, the housing material must have high mechanical strength to withstand the forces generated by the air flow and the weight of the adsorbent. This ensures that the filter maintains its shape and functionality over time, even under conditions of intensive use. The combination of a polymer with fiberglass can allow high mechanical strength and durability, being a lightweight and corrosion resistant part.

The filter of the present invention can take different shapes and measures, being preferably rectangular or square.

Preferably, each housing comprises a length of 300 to 500 mm long, a width of 100 to 300 mm, a height of 50 to 200 mm, and a thickness of 2 to 8 mm, preferably 5 mm. Said dimensions may be larger or smaller, depending on other embodiments.

The filter design should allow easy access for maintenance and cleaning. This includes the ability to disassemble and replace the housings or adsorbent without the need for special tools, which reduces system downtime and facilitates periodic maintenance.

Suction openings and/or extraction openings can be made in different ways.

According to a first embodiment, the suction and/or extraction openings take the form of orifices, each preferably with a diameter of 1 to 3 mm, and more preferably 2 mm. These dimensions may be larger or smaller, depending on other embodiments. Preferably, these holes are distributed in groups on the surface of the suction and extraction sides, thus creating identical suction and extraction areas along the surface of each side.

Preferably, the groups of holes are distributed inversely on one side with respect to the other, according to the position of the openings. Preferably the holes are distributed in each group forming rows and columns, or in staggered formation.

According to a second case of embodiment, the suction openings and/or the extraction openings are formed as open grids or openings, preferably placing a pre-filter blanket G4 (for coarse particles). In order to avoid both the release of the particles from the adsorbent filler and the dust that may come from it.

The suction openings and/or extraction openings can be rectangular or oval, allowing in the latter case to increase the size of the opening in the central part of the filter.

The filter may consist of one or more housings. Preferably, the filter is formed by a plurality of housings joined laterally, each of them being formed as a cartridge. The filter presents a waterproof feature.

According to different preferred embodiments, the filter may have a rectangular configuration consisting of two, three or four laterally joined housings, thus offering multiple combinations to suit various filtration needs. As an example, each of these housings or cartridges has the following characteristics:
- Weight: 1.80kg approximately;
- External dimensions in mm: 390 x 182 x 55;
- Material: Polypropylene of 5 mm thickness;
- Maximum adsorbent load: 1.4kg approximately.
- No. of carbon mesh: 6x12 mesh (from 3.35 mm to 1.68mm particle size).

Preferably, the adsorbent used by the filter of the present invention in any of its embodiments is active carbon, zeolite or alumina.

Preferably, the air filter of the present invention is an air filter for laboratory cabinets, such as gas filtration cabinets.

The present invention also has as its object a laboratory cabinet, such as a gas filtration cabinet, comprising one or more air filters according to any of the embodiments described above.

### Brief description of the drawings

Then, we begin to describe in a very brief way a series of drawings that help to better understand the invention and that are expressly related to several embodiments of this invention that are presented as non-limiting examples of it.
Figure 1 represents a perspective view of a conventional air filter.
Figure 2 represents a longitudinal section of the filter in Fig. 1.
Figure 3 depicts a perspective view of an air filter known in the state of the art.
Figure 4 represents a longitudinal section of the filter in Fig. 3.
Figure 5 represents a perspective view of an air filter according to the present invention in accordance with a first preferred embodiment.
Figure 6 represents a longitudinal section of the filter in Fig. 5.
Figure 7 represents a perspective view of an air filter according to the present invention in accordance with a second preferred embodiment.
Figure 8 represents a cross-section of the filter in Fig. 7.
Figure 9 represents a perspective view of an air filter according to the present invention in accordance with a third preferred embodiment.
Figure 10 represents a cross section of the filter in Fig. 9.
Figure 11 represents a perspective view of an air filter according to the present invention in accordance with a fourth preferred embodiment.
Figure 12 represents a cross section of the filter in Fig. 11.
Figure 13 represents a perspective view of an air filter according to the present invention in accordance with a fifth preferred embodiment.
Figure 14 represents a cross section of the filter in Fig. 13.
Figure 15 represents a perspective view of an air filter according to the present invention in accordance with a sixth preferred embodiment.
Figure 16 represents a cross section of the filter in Fig. 15.
Figure 17 represents a perspective view of an air filter according to the present invention in accordance with a seventh preferred embodiment.
Figure 18 depicts a front view of the suction side of the air filter of Fig. 17.
Figure 19 depicts a front view of the air filter extraction side of Fig. 17.

### Detailed description of the invention

Figures 1 and 2 represent a perspective view and a longitudinal section of a conventional air filter (1'). This filter (1') is formed by a housing (2') configured to contain inside a compact filling of an adsorbent (C), such as activated carbon. The housing (2') presents a suction side (3') and an extraction side (4'). The suction side (3') has a distribution of suction openings (31') that allows an air flow (F) to enter the filter (1'). The extraction side (4') has a distribution of extraction openings (41') that allows the air flow (F) to escape from the filter (1'). As can be seen, the suction openings (31') of the suction side (3') are distributed exactly the same and symmetrical to the extraction openings (41') of the extraction side (4').

Thus, being the engine of the cab located on the extraction side (4') in the central part of the filter (1'), it is observed how most of the flow (F) passes through the central part of the filter (1'), without passing through the ends or lateral areas of the filter. This leads to the formation of airtight zones (E) inside the filter (1'), where the adsorbent (C) is hardly used, since the air flow (F) does not reach them. So that the adsorbent (C) fills much earlier in the central part of the filter (1') than in the rest of the areas. This involves the replacement of the filter (1') even though much of the adsorbent (C) around that central part is still in perfect condition to continue with the adsorption functions. So, in the filter (1') of Figs. 1 and 2, a large amount of adsorbent (C) is discarded, barely unused, because the break point is reached prematurely in the central part of the filter (1'). That is, in a non-homogeneous way.

According to the tests performed for the conventional filter (1') in Figs. 1 and 2, the filter (1') becomes saturated much earlier, because the central part becomes clogged faster than the parts farther away from it.

According to AFNOR NFX 15-211, the test methodology consists of maintaining 200 ppm of isopropanol at all times in the volume of a cabinet by means of controlled evaporation thanks to a peristaltic pump that supplies the precise flow to achieve this concentration in the environment. The experiment is considered to have come to an end when 1% of the allowed TLV (environmental limit value) is detected at the exit of the cabin (TLV of isopropanol is 200ppm, therefore, the experiment ends when at the exit you have 2ppm). The concentration at the outlet is measured just at the outlet of the cabinet using a PID (Photoionization Detector) calibrated for organic compounds. The obtained results are summarized in the following tables:

| Table I: Conventional filter - Assay 1 Standard Load (7kg approximately of activated charcoal) | | | |
|---|---|---|---|
| Measurement | Actual value (ppm) | Total evaporated mass (g) | Assay time (min) |
| 1 | 0 | 0 | 0 |
| 10 | 0 | 200 | 154 |
| 20 | 1.38 | 336 | 267 |
| 24 | 2.07* | 380* | 303* |
| 28 | 4.14* | 420* | 337* |

| Table II: Conventional filter - Assay 2 Standard Load (7kg approximately of activated charcoal) | | | |
|---|---|---|---|
| Measurement | Actual value (ppm) | Total evaporated mass (g) | Assay time (min) |
| 1 | 0 | 0 | 0 |
| 10 | 0 | 196 | 162 |
| 15 | 1.61 | 316 | 259 |
| 16 | 2.30* | 334* | 273* |
| 19 | 4.14* | 386* | 318* |

Thus, according to the tests carried out on the conventional filter (1'), it is saturated between 273 and 303 minutes.

Figures 3 and 4 represent a perspective view and a longitudinal section of another air filter (1") known in the state of the art.

As can be seen, the air filter (1") comprises a casing (2") configured to contain within it an adsorbent (C), such as activated carbon. The housing (2') presents a suction side (3') and an extraction side (4'). The suction side (3) has a distribution of suction openings (31") that allows an air flow (F) to enter the filter (1"). The extraction side (4") has a distribution of extraction openings (41") that allows the air flow (F') to escape from the filter (1"). The suction openings (31") on the suction side (3") are distributed in reverse to the extraction openings (41") on the extraction side (4") to redirect the air flow (F) inside the filter (1").

This allows directing the air flow (F) through the adsorbent filler to increase the contact area between the adsorbent and the adsorbate; and decreasing its velocity so that the contact time between the filter coating (1") and the air flow (F) is much longer, which results in a greater optimization of the adsorbent, i.e., it considerably increases the residence time of the flow within the filter bed.

However, there is also the formation of airtight zones (E) inside the filter (1 "), where the adsorbent (C) is hardly used, since the air flow (F) does not reach them. This entails the replacement of the filter (1") even though part of the adsorbent (C) closer to the ends or lateral areas of the filter is still in perfect condition to continue with the adsorption functions. So, in the filter (1") of Figs. 3 and 4 also discard a quantity of adsorbent (C) that is hardly unused.

According to the tests carried out for the filter (1") in Figs. 3 and 4, it is observed that the filter (1") saturates later than the conventional filter (1').

The test methodology also consists, according to AFNOR NFX 15-211, in maintaining 200 ppm of isopropanol at all times in the volume of a cabin by means of controlled evaporation thanks to a peristaltic pump that supplies the precise flow to achieve this concentration in the environment. The experiment is considered to have come to an end when 1% of the allowed TLV (environmental limit value) is detected at the exit of the cabin (TLV of isopropanol is 200ppm, therefore, the experiment ends when at the exit you have 2ppm). The concentration at the outlet is measured just at the outlet of the cabinet using a PID (Photoionization Detector) calibrated for organic compounds. The obtained results are summarized in the following tables:

| Table III: Filter of the present invention - Standard Load (7kg approximately of activated charcoal) | | | |
|---|---|---|---|
| Measurement | Actual value (ppm) | Total evaporated mass (g) | Assay time (min) |
| 1 | 0 | 0 | 0 |
| 10 | 0 | 216 | 181 |
| 20 | 0 | 356 | 276 |
| 30 | 0.92 | 514 | 390 |
| 33 | 2.30* | 556* | 420* |
| 36 | 4.60* | 588* | 445* |

| Table IV: Filter of the present invention - Maximum Load (9kg approximately of activated charcoal) | | | |
|---|---|---|---|
| Measurement | Actual value (ppm) | Total evaporated mass (g) | Assay time (min) |
| 1 | 0 | 0 | 0 |
| 10 | 0 | 234 | 176 |
| 20 | 0 | 482 | 374 |
| 30 | 1.38 | 658 | 511 |
| 31 | 2.07* | 670* | 521 * |
| 34 | 4.14* | 712* | 553* |

Thus, according to the tests carried out on the filter (1") of Figs. 3 and 4, it saturates at around 420 minutes for a standard adsorbent load (C), and at around 521 minutes for a maximum adsorbent load (C). That is, it saturates later than a conventional filter (1') with the same standard load.

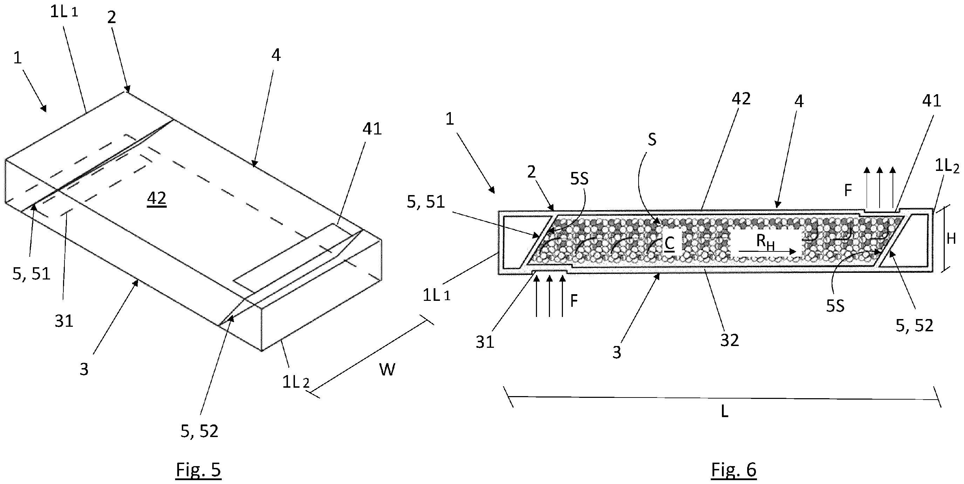

Figures 5 and 6 represent a perspective view and a longitudinal section of the air filter (1) of the present invention according to a first embodiment thereof. As can be seen, the filter (1) comprises a housing (2) with an inner space (S) configured to contain an adsorbent (C). The filter (1) has:
- a suction side (3) with a suction opening (31) that allows an air flow (F) to enter the filter (1); and
- an extraction side (4) with an extraction opening (41) allowing the air flow (F) to exit out of the filter (1).

The suction side (3) has suction separation zones (32) that prevent the passage of the air flow (F). Similarly, the extraction side (4) has extraction separation zones (42) that impede the passage of the air flow (F).

The filter (1) is characterized in that the inner space (S) is delimited by end deflecting means (5), configured to redirect the air flow (F) from the suction opening (31) towards the extraction opening (41).

According to the present example, the deflecting means (5) are arranged at a first longitudinal end (1L₁) and at a second longitudinal end (1L₂) of the filter (1), i.e., along the width (W) of the filter (1) or across the width of the filter (1).

The end deflecting means (5) comprise:
- a first transverse baffle (51) arranged along the first longitudinal end (1L₁) of the filter (1); and
- a second transverse baffle (52) arranged along the second longitudinal end (1 L₁) of the filter (1).

The absorbent (C) is thus contained solely within the inner space (S) of the housing (2), the end deflecting means (5, 51, 52) occupying an absorbent-free zone (C). So that the longitudinal ends (1Li, 1L₂) are free of absorbent (C).

The end deflecting means (5, 51, 52) are designed to direct and distribute the air flow (F) evenly across the adsorbent (C), eliminating areas where air could become stagnant and not interact with said adsorbent material (C).

The end deflecting means (5, 51, 52) thus prevent the formation of air-tight zones (E) inside the filter (1), where the adsorbent (C) is not effectively used.

The suction opening (31) and the extraction opening (41) are arranged parallel to the first transverse baffle (51) and the second transverse baffle (52) respectively.

The first transverse baffle (51) is arranged next to the suction side (3) adjacent to the suction opening (31). The first transverse baffle (51) is positioned just downstream of the suction opening (31). Its main function is to guide the incoming air flow (F) so that it is evenly distributed across the adsorbent (C). This ensures that the entire volume of air passes through the adsorbent material (C), maximizing contact and adsorption efficiency.

The second transverse baffle (52) is arranged next to the extraction side (4) adjacent to the extraction opening (41). The second transverse baffle (52) is positioned just before the extraction opening (41). Its function is to redirect the outgoing air flow (F), ensuring that a constant, turbulence-free flow is maintained. This helps maintain system efficiency and ensures that the air has adequately passed through the adsorbent (C) before exiting the filter (1).

The suction opening (31) of the suction side (3) is arranged symmetrically opposite to the extraction opening (41) of the extraction side (4) to redirect the air flow (F) inside the filter (1). In this way, the air flow (F) is redirected inside the filter (1) increasing its horizontal path (R_{H}) through the filter (1) to stay longer inside the filter and pass through both the central part and the perimeter or lateral part of the adsorbent bed.

The end deflecting means (5, 51, 52) form an integral part of the housing (2), being part of its structure.

The end deflecting means (5, 51, 52) have at least one end deflecting surface (5S) in contact with the adsorbent (C), which has a straight or curved configuration and which is arranged obliquely to the suction side (3) and to the extraction side (4).

Preferably, the housing (2) comprises a length (L) of 300 to 500 mm long, a width (W) of 100 to 300 mm, a height (H) of 50 to 200 mm, and a thickness (e) of 2 to 8 mm, preferably 5 mm. Said dimensions may be larger or smaller, depending on other embodiments.

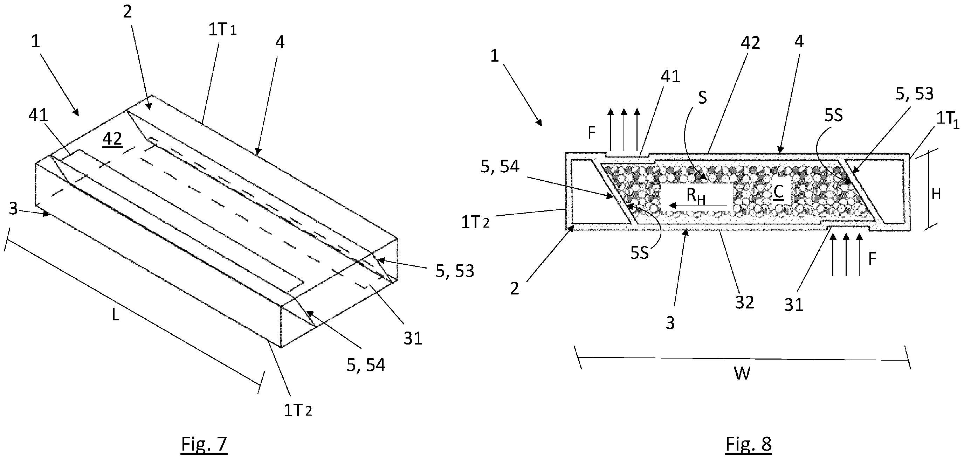

Figures 7 and 8 represent a perspective view and a cross section of the air filter (1) of the present invention according to a second embodiment thereof. As can be seen, the filter (1) comprises a housing (2) with an inner space (S) configured to contain an adsorbent (C). The filter (1) has:
- a suction side (3) with a suction opening (31) that allows an air flow (F) to enter the filter (1); and
- an extraction side (4) with an extraction opening (41) allowing the air flow (F) to exit out of the filter (1).

The suction side (3) has suction separation zones (32) that prevent the passage of the air flow (F). Similarly, the extraction side (4) has extraction separation zones (42) that impede the passage of the air flow (F).

The filter (1) is characterized in that the inner space (S) is delimited by end deflecting means (5), configured to redirect the air flow (F) from the suction opening (31) towards the extraction opening (41).

According to the present example, the deflecting means (5) are arranged at a first transverse end (1T₁) and at a second transverse end (1T₂) of the filter (1), i.e. along the length (L) of the filter (1) or along the length of the filter (1).

The end deflecting means (5) comprise:
- a first longitudinal baffle (53) arranged along the first transverse end (1T₁) of the filter (1); and
- a second longitudinal baffle (54) arranged along the second transverse end (1T₂) of the filter (1).

The absorbent (C) is thus contained solely within the inner space (S) of the housing (2), the end deflecting means (5, 53, 54) occupying an absorbent-free zone (C). So that the transverse ends (1T₁,1T₂) are free of absorbent (C).

The end deflecting means (5, 53, 54) are designed to direct and distribute the air flow (F) evenly across the adsorbent (C), eliminating areas where air could become stagnant and not interact with said adsorbent material (C).

The end deflecting means (5, 53, 54) thus prevent the formation of air-tight zones (E) inside the filter (1), where the adsorbent (C) is not effectively used.

The suction opening (31) and the extraction opening (41) are arranged parallel to the first longitudinal baffle (53) and the second longitudinal baffle (54) respectively.

The first longitudinal baffle (53) is arranged next to the suction side (3) adjacent to the suction opening (31). The first longitudinal baffle (53) is positioned just downstream of the suction opening (31). Its main function is to guide the incoming air flow (F) so that it is evenly distributed across the adsorbent (C). This ensures that the entire volume of air passes through the adsorbent material (C), maximizing contact and adsorption efficiency.

The second longitudinal baffle (54) is arranged next to the extraction side (4) adjacent to the extraction opening (41). The second longitudinal baffle (54) are positioned just before the extraction opening (41). Its function is to redirect the outgoing air flow (F), ensuring that a constant, turbulence-free flow is maintained. This helps maintain system efficiency and ensures that the air has adequately passed through the adsorbent (C) before exiting the filter (1).

The suction opening (31) of the suction side (3) is arranged symmetrically opposite to the extraction opening (41) of the extraction side (4) to redirect the air flow (F) inside the filter (1). In this way, the air flow (F) is redirected inside the filter (1) increasing its horizontal path (R_{H}) through the filter (1) to stay longer inside the filter and pass through both the central part and the perimeter or lateral part of the adsorbent bed.

The end deflecting means (5, 53, 54) form an integral part of the housing (2), being part of its structure.

The end deflecting means (5, 53, 54) have at least one end deflecting surface (5S) in contact with the adsorbent (C), which has a straight or curved configuration and which is arranged obliquely to the suction side (3) and to the extraction side (4).

Preferably, the housing (2) comprises a length (L) of 300 to 500 mm long, a width (W) of 100 to 300 mm, a height (H) of 50 to 200 mm, and a thickness (e) of 2 to 8 mm, preferably 5 mm. Said dimensions may be larger or smaller, depending on other embodiments.

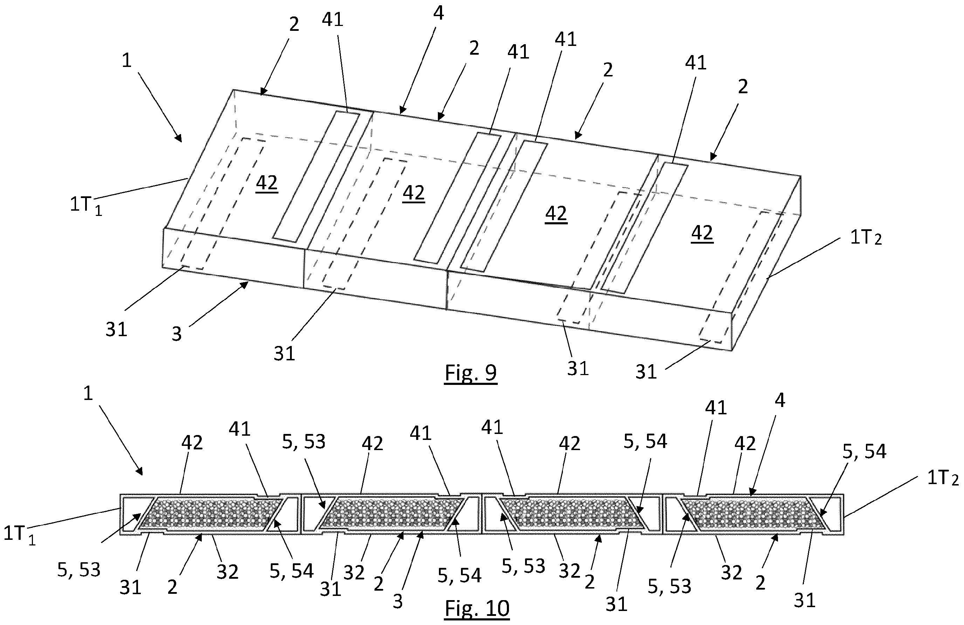

Figures 9 and 10 represent a perspective view and a cross-section of the air filter (1) of the present invention according to a third preferred embodiment. In this case, the filter (1) consists of four housings (2) as described in Figures 7 and 8, joined laterally. Each housing (2) is shaped like a cartridge.

The two housings (2) on the left are arranged symmetrically with respect to the two housings (2) on the right. The suction openings (31) are located further away from the central part of the filter (1), while the extraction openings (41) are located closer to said central part and, therefore, closer to the cabin motor, normally located over the extraction side (4) in the central part of the filter (1).

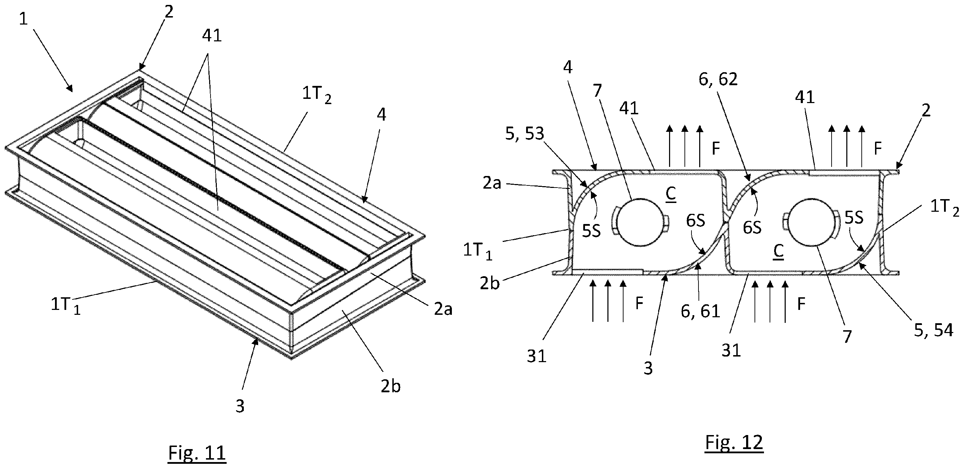

Figures 11 and 12 represent a perspective view and a cross-section of the air filter (1) of the present invention according to a fourth preferred embodiment.

According to the present embodiment, the inner space (S) is further delimited by intermediate deflecting means (6), configured to redirect the air flow (F) towards at least one exhaust opening (41). Said intermediate deflecting means (6) are arranged between the first transverse end (1T₁) and the second transverse end (1T₂) of the filter (1).

The intermediate deflecting means (6) comprise a first intermediate baffle (61) proximate to the suction side (3) and contiguous to a suction opening (31). The intermediate deflecting means (6) are positioned just downstream of the suction openings (31). Its main function is to guide the incoming air flow (F) so that it is evenly distributed across the adsorbent (C). This ensures that the entire volume of air passes through the adsorbent material (C), maximizing contact and adsorption efficiency.

The intermediate deflecting means (6) comprise a second intermediate baffle (62) proximate to the extraction side (4) and contiguous to an extraction opening (41). The intermediate deflecting means (6) are positioned just before the extraction openings (41). Its function is to redirect the outgoing air flow (F), ensuring that a constant, turbulence-free flow is maintained. This helps maintain system efficiency and ensures that the air has adequately passed through the adsorbent (C) before exiting the filter (1).

The absorbent (C) is thus contained solely within the inner space (S) of the housing (2), the intermediate deflecting means (6, 61, 62) occupying an absorbent-free zone (C) between the transverse ends (1T₁,1T₂).

The intermediate deflecting means (6, 61, 62) are designed to direct and distribute the air flow (F) evenly across the adsorbent (C), eliminating areas where air could become stagnant and not interact with said adsorbent material (C).

The intermediate deflecting means (6, 61, 62) thus prevent the formation of air-tight zones (E) inside the filter (1), where the adsorbent (C) is not effectively used.

The suction side (3) has two suction openings (31). The extraction side (4) also has two extraction openings (41).

The suction openings (31) and the extraction openings (41) are arranged parallel to the intermediate baffles (61, 62). The suction openings (31) and the extraction openings (41) are configured as grids.

The suction openings (31) on the suction side (3) are distributed in reverse to the extraction openings (41) on the extraction side (4) to redirect the air flow (F) inside the filter (1). In this way, the air flow (F) is redirected inside the filter (1) increasing its horizontal path (R_{H}) through the filter (1) to stay longer inside the filter and pass through both the central part and the perimeter or lateral part of the adsorbent bed.

The intermediate deflecting means (6, 61, 62) form an integral part of the housing (2), being part of its structure.

The intermediate deflecting means (6, 61, 62) have at least one extreme deflecting surface (6S) in contact with the adsorbent (C), which has a curved configuration and is arranged obliquely to the suction side (3) and the extraction side (4).

The housing (2) is formed by two half-bodies (2a, 2b) facing each other and joined perimetrically. The housing (2) comprises refill holes (7) enabled to allow loading of the adsorbent (C).

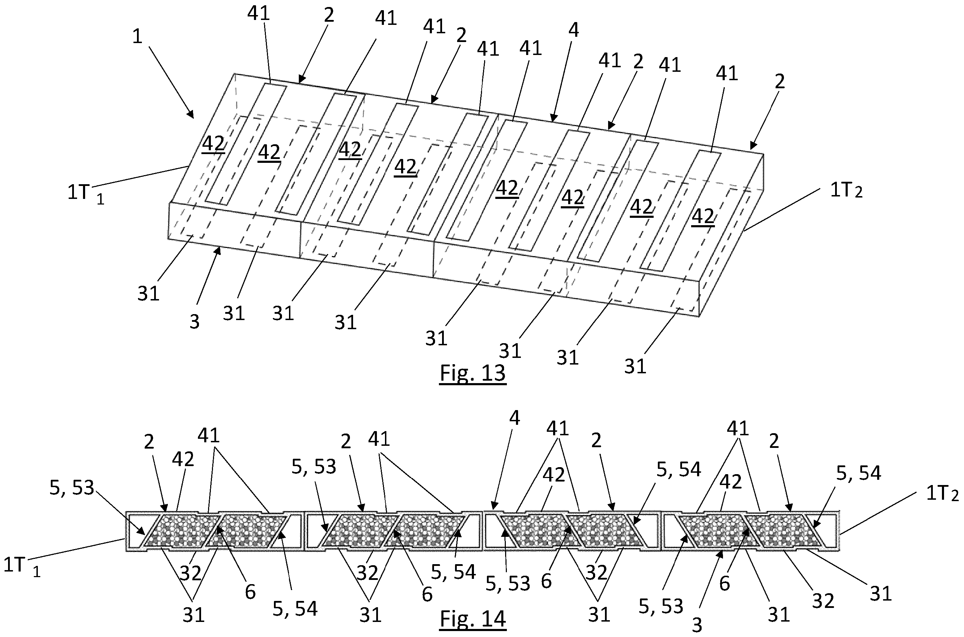

Figures 13 and 14 represent a perspective view and a cross-section of the air filter (1) of the present invention according to a fifth preferred embodiment. In this case, the filter (1) consists of four housings (2) as described in Figures 11 and 12, joined laterally. Each housing (2) is shaped like a cartridge.

The two housings (2) on the left are arranged symmetrically with respect to the two housings (2) on the right. The suction openings (31) are located further away from the central part of the filter (1), while the extraction openings (41) are located closer to said central part and, therefore, closer to the cabin motor, normally located over the extraction side (4) in the central part of the filter (1).

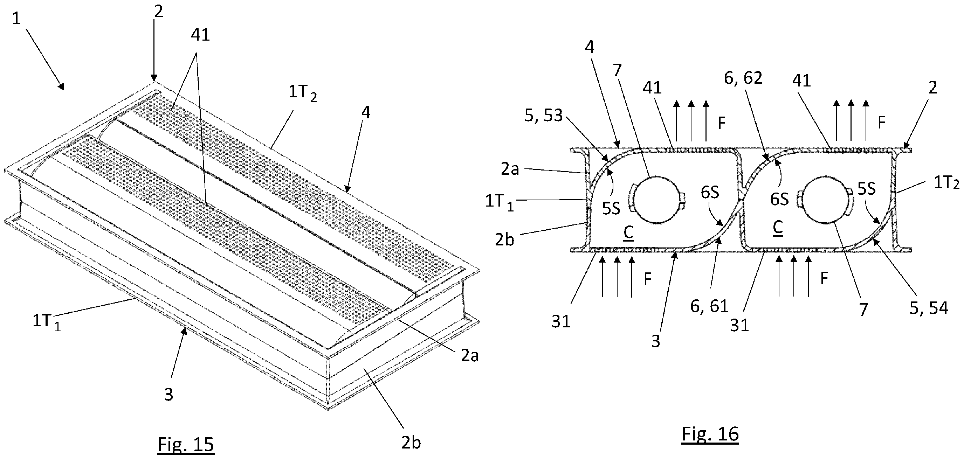

Figures 15 and 16 represent a perspective view and a cross-section of the air filter (1) of the present invention according to a sixth preferred embodiment. This example is like Figures 11 and 12, with the difference that, in this case, the suction openings (31) and the extraction openings (41) are configured as orifices.

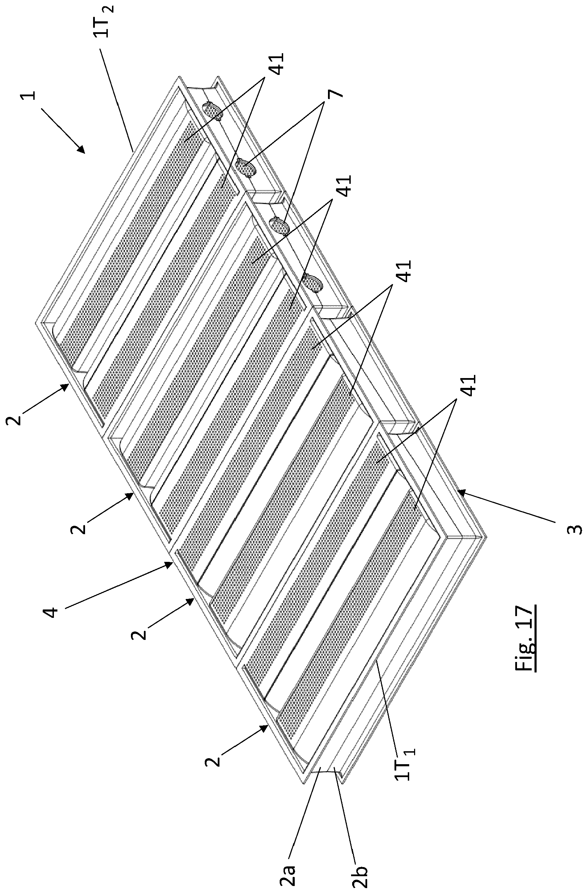

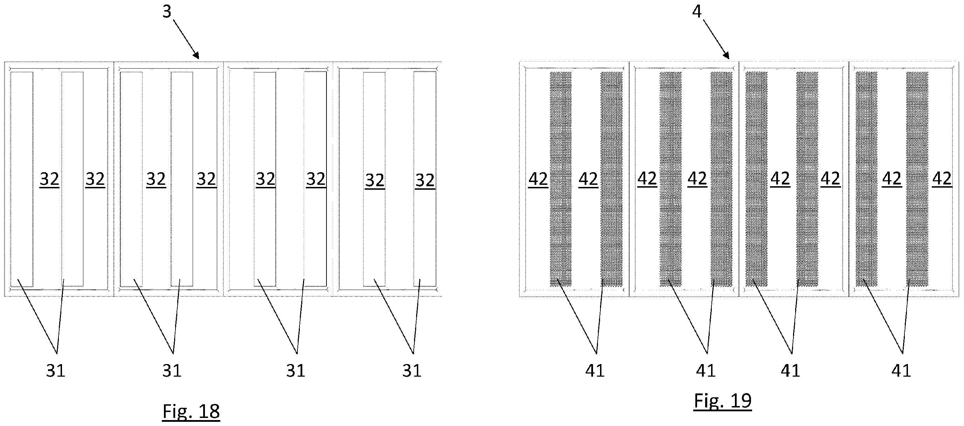

Figures 17 to 19 depict various views of the air filter (1) of the present invention according to a seventh preferred embodiment. In this case, the filter (1) consists of four housings (2) as shown in Figures 15 and 16, joined laterally. Each housing (2) is shaped like a cartridge.

The two housings (2) on the left are arranged symmetrically with respect to the two housings (2) on the right. The suction openings (31) are located further away from the central part of the filter (1), while the extraction openings (41) are located closer to said central part and, therefore, closer to the cabin motor, normally located over the extraction side (4) in the central part of the filter (1).

According to the tests carried out for the filter (1) of Figs. 17 to 19, said filter (1) has a high efficiency, avoids the formation of air-tight zones (E) inside the filter (1) and requires less activated carbon loading than the filters (1', 1") of Figs. 1 a 4.

The test methodology also consists, according to AFNOR NFX 15-211, in maintaining 200 ppm of isopropanol at all times in the volume of a cabin by means of controlled evaporation thanks to a peristaltic pump that supplies the precise flow to achieve this concentration in the environment. The experiment is considered to have come to an end when 1% of the allowed TLV (environmental limit value) is detected at the exit of the cabin (TLV of isopropanol is 200ppm, therefore, the experiment ends when at the exit you have 2ppm). The concentration at the outlet is measured just at the outlet of the cabinet using a PID (Photoionization Detector) calibrated for organic compounds. The obtained results are summarized in the following tables:

| Table V: Filter of the present invention - Test 1 Standard load (approx. 5.50 kg of activated carbon) | | | |
|---|---|---|---|
| Measurement | Actual value (ppm) | Total evaporated mass (g) | Assay time (min) |
| 1 | 0 | 0 | 0 |
| 10 | 0 | 80 | 60 |
| 20 | 0 | 156 | 120 |
| 30 | 0.46 | 222 | 180 |
| 40 | 1.38 | 284 | 240 |
| 50 | 2.30* | 362* | 300* |
| 52 | 4.14* | 434* | 320* |

| Table VI: Filter of the present invention - Test 2 Maximum load (approx. 5,84 kg activated carbon) | | | |
|---|---|---|---|
| Measurement | Actual value (ppm) | Total evaporated mass (g) | Assay time (min) |
| 1 | 0 | 0 | 0 |
| 10 | 0 | 86 | 60 |
| 20 | 0 | 162 | 120 |
| 30 | 0.46 | 238 | 180 |
| 40 | 1.38 | 312 | 240 |
| 50 | 2.30* | 394* | 300* |
| 57 | 4.14* | 466* | 336* |

Thus, according to the tests carried out on the filter (1) of the present invention, it saturates after 300 minutes for a reduced adsorbent load (C). That is, with a lower load than that used in the conventional filter (1), a higher retention capacity is achieved in a similar period. In addition, the formation of air-tight zones (E) inside the filter (1) is prevented, contrary to what happens in the filters (1', 1") of Figs. 1 to 4.

As an example, the filter (1) of Figs. 17 to 19 has the following characteristics:
- Dimensions (length x width x height): 730 x 390 x 55 mm
- Weight: 2.50 kg (without charcoal)
- Material: Polypropylene of 1,5 mm thickness;
- Maximum carbon load per cell or casing: 1.4kg approximately.
- N. Carbon mesh: 6x12 mesh (Particle size 1.68 - 3.35 mm)
- Surface area 1100m²/g
- Density 470 kg/m³

## Claims

1. Air filter, comprising at least one housing (2) with an inner space (S) configured to contain an adsorbent (C) and featuring:
- a suction side (3) with one or more suction openings (31) that allow an air flow (F) to enter the filter (1); and
- an extraction side (4) with one or more extraction openings (41) allowing the air flow (F) to be exhausted out of the filter (1);
said filter (1) **characterized in that** the inner space (S) is delimited by end deflecting means (5), configured to redirect the air flow (F) towards at least one extraction opening (41), where said end deflecting means (5) are arranged in:
- a first longitudinal end (1L₁) and/or at a second longitudinal end (1L₂) of the filter (1); and/or in
- a first transverse end (1T₁) and/or at a second transverse end (1T₂) of the filter (1).

2. Air filter according to claim 1, **characterized in that** the end deflecting means (5) comprise:
- a first transverse baffle (51) arranged along the first longitudinal end (1L₁) of the filter (1); and/or
- a second transverse baffle (52) arranged along the second longitudinal end (1L₁) of the filter (1).

3. Air filter according to any one of claims 1 to 2, **characterized in that** the end deflecting means (5) comprise:
- a first longitudinal baffle (53) arranged along the first transverse end (1T₁) of the filter (1); and/or
- a second longitudinal baffle (54) arranged along the second transverse end (1T₂) of the filter (1).

4. Air filter according to any one of claims 1 to 3, **characterized in that** the end deflecting means (5) are arranged:
- proximal to the suction side (3) contiguous to a suction opening (31); and/or
- close to the extraction side (4) adjacent to an extraction opening (41).

5. Air filter according to any of claims 1 to 4, **characterized in that** the end deflecting means (5) form an integral part of the housing (2) or independent elements attached thereto.

6. Air filter according to any of claims 1 to 5, **characterized in that** the end deflecting means (5) present at least one extreme deflecting surface (5S) in contact with the adsorbent (C), which presents a straight or curved configuration and which is arranged obliquely to the suction side (3) and to the extraction side (4).

7. Air filter according to any of claims 1 to 6, **characterized in that** the inner space (S) is additionally delimited by intermediate deflecting means (6), configured to redirect the air flow (F) towards at least one extraction opening (41), wherein said intermediate deflecting means (6) are arranged between:
- the first longitudinal end (1L₁) and the second longitudinal end (1L₂) of the filter (1); and/or between
- the first transverse end (1T₁) and the second transverse end (1T₂) of the filter (1).

8. Air filter according to claim 7, **characterized in that** the intermediate deflecting means (6) comprise a first intermediate baffle (61) close to the suction side (3) and contiguous to a suction opening (31).

9. Air filter according to any of claims 7 to 8, **characterized in that** the intermediate deflecting means (6) comprise a second intermediate baffle (62) close to the extraction side (4) and contiguous to an extraction opening (41).

10. Air filter according to any of claims 7 to 9, **characterized in that** the intermediate deflecting means (6) form an integral part of the housing (2) or independent elements attached thereto.

11. Air filter according to any of claims 7 to 10, **characterized in that** the intermediate deflecting means (6) present at least one intermediate deflecting surface (6S) in contact with the adsorbent (C), which presents a straight or curved configuration and which is arranged obliquely to the suction side (3) and to the extraction side (4).

12. Air filter according to any one of claims 1 to 11, **characterized in that** the suction openings (31) of the suction side (3) are distributed symmetrically inverse to the extraction openings (41) of the extraction side (4) to redirect the air flow (F) through the interior of the filter (1).

13. Air filter according to any of claims 1 to 12, **characterized in that** the housing (2) is formed by two half bodies (2a, 2b), each comprising the suction side (3) or the extraction side (4), faced with each other and united perimetrally.

14. Air filter according to any one of claims 1 to 13, **characterized in that** the housing (2) comprises a length (L) of 300 to 500 mm, a width (W) of 100 to 300 mm, a height (H) of 50 to 200 mm, and a thickness (e) of 2 to 8 mm.

15. Air filter according to any of claims 1 to 14, **wherein** the air filter (1) is for laboratory cabinets, such as gas filtration cabinets.

16. Laboratory cabinet, **wherein** an air filter is comprised (1) according to any of the previous claims 1 to 15.
